# EUROPEAN PATENT APPLICATION

(11) **EP 2 782 061 A1**
(43) Date of publication of application: **24.09.2014**
(21) Application number: 14160949.5
(22) Date of filing: 20.03.2014
(51) Int. Cl.: G06Q 30/06, G06Q 50/22

(54) **System and method for recommending health management device using mobile device connectivity information**

(30) Priority: 20.03.2013 US 201361803564 P; 19.07.2013 KR 20130085679
(71) Applicant: Samsung Electronics Co., Ltd., Suwon-si Gyeonggi-do 443-742 (KR)
(72) Inventor: Park, Jeong-je, Gyeonggi-do (KR); Lee, Kwang-hyeon, Seoul (KR); Kim, Ji-eun, Gyeonggi-do (KR); Chang, Seok-jin, Gyeonggi-do (KR)
(74) Representative: Grünecker, Kinkeldey, Stockmair & Schwanhäusser

(57) **Abstract**

A method for recommending a health management device using connectivity information of a client device includes: receiving the connectivity information of the client device; recommending health management device, which is able to be connected to the client device, based on the received connectivity information and information about the health management device; and transmitting information of the recommended health management device to the client device.

## Description

### CROSS-REFERENCE TO RELATED APPLICATIONS

This application claims priority from U.S. Provisional Patent Application No. 61/803,564, filed on March 20, 2013, and Korean Patent Application No. 10-2013-0085679, filed on July 19, 2013, in the Korean Intellectual Property Office, the disclosures of which are incorporated herein in their entireties by reference.

### BACKGROUND

### 1. Field

Apparatuses and methods consistent with exemplary embodiments relate to recommending a health management device using connectivity information regarding a client device, and more particularly, to recommending a health management device that may be connected to a user device based on connection methods supported by the client device and health information of a user.

### 2. Description of the Related Art

Along with developments in communication technologies and miniaturization of communication modules, communication functions tend to be integrated in all electronic devices.

Health management devices with communication functions are being developed and sold, and such a health management device may be connected to a client device, e.g., a mobile phone of a user, to perform functions that cannot be performed by the health management device itself by accessing an external server via the client device or using an application or a program designed for the client device.

However, if a user is not familiar with communication functions integrated in his or her client device, the user may mistakenly purchase a health management device that cannot be connected to the client device or may have to check separate information for determining communication functions integrated in the client device. Particularly, the major consumer group using health management devices is senior citizens, and there are difficulties for senior citizens to determine their health conditions and information regarding their client devices and to search for and select suitable health management devices based on the information.

### SUMMARY

Exemplary embodiments address at least the above problems and/or disadvantages and other disadvantages not described above. Also, the exemplary embodiments are not required to overcome the disadvantages described above, and may not overcome any of the problems described above.

One or more exemplary embodiments include a system and a method for recommending only health management devices that may be connected to a client device and is suitable for health condition of a user to reduce purchase inconvenience and preventing purchase of a wrong health management device.

Additional aspects will be set forth in part in the description which follows and, in part, will be apparent from the description, or may be learned by practice of the presented embodiments.

According to one or more exemplary embodiments, a method that a health management device recommending server recommends health management devices by using connectivity information regarding a client device, the method includes receiving the connectivity information regarding the client device from the client device; recommending health management devices, which are able to be connected to the client device, based on the received connectivity information and information regarding at least one health management device; and transmitting information regarding the recommended health management devices to the client device.

The connectivity information includes at least one from among communication methods and communication protocols supported by the client device and communication protocols compatible with the communication protocols.

The communication methods includes at least one from among Wi-Fi communication, Bluetooth communication, universal serial bus (USB) communication, near field communication (NFC), ZigBee communication, ultra wide band (UWB) communication, and ANT+ communication.

The receiving of the connectivity information further includes receiving user's health information from at least one from between the client device and a health information providing server, and, in the recommending of the health management devices, health management devices related to health condition of the user are recommended from among the health management devices that are able to be connected to the client device, based on the received health information.

The receiving of the connectivity information further includes receiving information regarding preference of the user with respect to health management devices, and, in the recommending of the health management devices, the health management devices are recommended based on the health information, the preference information, and the connectivity information.

The preference information includes at least one from among body parts to which health management devices are to be attached, information regarding a user's preference of brands of health management devices, and a history of purchasing health management devices.

The information regarding at least one health management device include information regarding only health management devices with verified connectivity with the client device.

According to one or more exemplary embodiments, a method that a client device recommends health management devices, the method includes transmitting connectivity information regarding the client device to a server; receiving information regarding health management devices recommended by the server from the server; and displaying a list of the recommended health management devices based on the received information regarding the recommended health management devices, wherein the recommended health management devices are devices that are able to be connected to the client device.

The connectivity information includes at least one from among communication methods and communication protocols supported by the client device and communication protocols compatible with the communication protocols.

The communication methods includes at least one from among Wi-Fi communication, Bluetooth communication, universal serial bus (USB) communication, near field communication (NFC), ZigBee communication, ultra wide band (UWB) communication, and ANT+ communication.

The recommended health management devices are determined based on user's health information transmitted from at least one from between the client device and a health information providing server and health condition of the user.

The transmitting of the connectivity information further includes transmitting information regarding preference of the user with respect to health management devices, and the recommended health management devices are recommended based on the health information, the preference information, and the connectivity information.

The preference information includes at least one from among body parts to which health management devices are to be attached, information regarding a user's preference of brands of health management devices, and a history of purchasing health management devices.

According to one or more exemplary embodiments, a server that recommends health management devices by using connectivity information regarding a client device, the server includes a receiver, which receives the connectivity information regarding the client device from the client device; a controller, which recommends health management devices, which are able to be connected to the client device, based on the received connectivity information and information regarding health management devices; and a transmitter, which transmits information regarding the recommended health management devices to the client device.

According to one or more exemplary embodiments, a client device that recommends health management devices by using connectivity information regarding the client device, the client device includes at least one communicator capable of communicating with an external device; a transmitter, which transmits connectivity information regarding the client device to a server; a health management device information receiver, which receives information regarding health management devices recommended by the server from the server; and a display, which displays a list of the recommended health management devices based on the received information regarding the recommended health management devices, wherein the recommended health management devices are recommended by the server based on the connectivity information and are devices that are able to be connected to the client device.

According to one or more exemplary embodiments, a method of recommending health management devices by using connectivity information regarding a client device, the method includes extracting the connectivity information regarding the client device; and recommending health management devices that are able to be connected to the client device based on the extracted connectivity information regarding the client device and information regarding at least one health management device.

The method further includes extracting user's health information from the client device, wherein, in the recommending of the health management devices, health management devices related to health condition of the user are recommended from among the health management devices that are able to be connected to the client device, based on the extracted health information.

The method further includes receiving user's health information from a health information providing server, wherein, in the recommending of the health management devices, health management devices related to health condition of the user are recommended from among the health management devices that are able to be connected to the client device, based on the received health information.

In the recommending of the health management devices, health management devices having relatively high priorities are recommended from among the health management devices that are able to be connected to the client device.

The method further includes requesting the information regarding at least one health management device to an external server; and receiving the information regarding at least one health management device from the external server.

The recommended health management devices are determined by an application being executed on the client device.

According to one or more exemplary embodiments, a client device that recommends health management devices by using connectivity information regarding the client device, the client device includes at least one communicator capable of communicating with an external device; and a controller, which recommends health management devices, which are able to be connected to the client device, based on the connectivity information regarding the client device and information regarding health management devices.

The client device may further include an extractor, which extracts user's health information from the client device, wherein the controller recommends only health management devices related to health condition of the user from among the health management devices, which are able to be connected to the client device, by further using the extracted health information.

The client device further includes a receiver, which receives user's health information from a health information providing server, wherein the controller for recommending health management devices recommends health management devices related to health condition of the user from among the health management devices that are able to be connected to the client device, based on the extracted health information.

The controller recommends health management devices having relatively high priorities from among the health management devices that are able to be connected to the client device.

The method further includes a transmitter, which requests the information regarding at least one health management device to an external server; and a receiver, which receives the information regarding at least one health management device from the external server.

The recommended health management devices are determined by an application being executed on the client device.

According to one or more exemplary embodiments, there is provided a computer-readable recording medium having recorded thereon a computer program for implementing the above-stated methods.

### BRIEF DESCRIPTION OF THE DRAWINGS

The above and/or other aspects will be more apparent by describing certain exemplary embodiments, with reference to the accompanying drawings, in which:
FIG. 1 is a diagram showing a health management device recommending system using health information regarding a user and connectivity information regarding a client device, according to an exemplary embodiment;
FIG. 2 is a block diagram showing a health management device recommending system using a user's health information and connectivity information regarding a client device according to an exemplary embodiment;
FIG. 3 is a diagram showing the detailed configuration of a health management device recommending server according to an exemplary embodiment;
FIG. 4 is a diagram showing the detailed configuration of a client device according to an exemplary embodiment;
FIG. 5 is a flowchart showing operations of the health management device recommending server according to an exemplary embodiment;
FIG. 6 is a flowchart showing a method that a client device requests a health management device recommending server to recommend health management devices and receives information regarding recommended health management devices from the health management device recommending server, according to an exemplary embodiment;
FIG. 7 is a detailed flowchart showing a method of recommending health management devices by using user's health information and connectivity information regarding a client device according to an exemplary embodiment:
FIG. 8 is a detailed flowchart showing a method of recommending health management devices using user's health information and connectivity information regarding a client device according to an exemplary embodiment;
FIG. 9 is a detailed flowchart of a method of recommending health management devices by using user's health information and connectivity information regarding the client device, according to an exemplary embodiment;
FIG. 10 is a detailed flowchart showing a method of recommending health management devices by using user's health information and connectivity information regarding the client device, according to an exemplary embodiment;
FIG. 11 is a diagram showing an example of tables including information regarding health management devices according to an exemplary embodiment;
FIG. 12 is a diagram showing an example of tables including users' health information according to an exemplary embodiment;
FIG. 13 is a diagram showing an example of screen images showing results of recommending health management devices by using user's health information and connectivity information regarding a client device, according to an exemplary embodiment;
FIG. 14 is a block diagram showing a health management device recommending system using connectivity information regarding a client device, according to an exemplary embodiment; and
FIG. 15 is a flowchart showing operations of the client device according to an exemplary embodiment.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

The terms used in the present specification are merely used to describe certain exemplary embodiments, and are not intended to be limiting. An expression used in the singular encompasses the expression of the plural, unless it has a clearly different meaning in the context. In the present specification, it is to be understood that the terms such as "including" or "having," etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

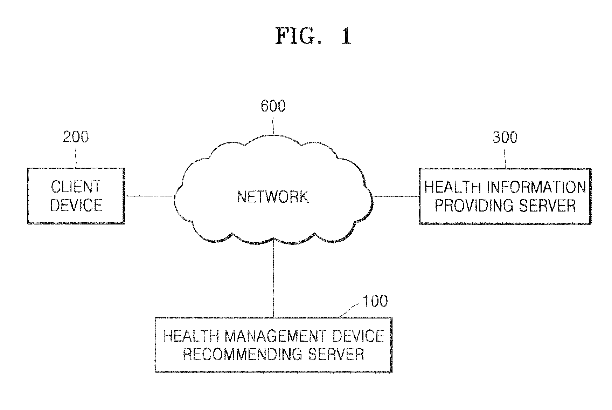

FIG. 1 is a diagram showing a health management device recommending system using health information regarding a user and connectivity information regarding a client device, according to an exemplary embodiment.

As shown in FIG. 1, the health management device recommending system using health information regarding a user and connectivity information regarding a client device includes health management device recommending server 100, a client device 200, a health information providing server 300, and a network 600.

The client device 200 may recommend a health management device suitable for a user.

The client device 200 may recommend a health management device based on connectivity information regarding the client device 200. The connectivity information regarding the client device 200 may include information necessary for connecting a health management device to a client device. For example, connectivity information regarding the client device may include information regarding close-distance communication methods, communication protocols, and communication profiles supported by the client device 200. The client device 200 may transmit the connectivity information regarding the client device 200 to the health management device recommending server 100. The client device 200 may receive information regarding health management device recommended based on the connectivity information regarding the client device 200 from the health management device recommending server 100.

Health management devices includes special purpose health management devices, such as a blood pressure gauge and a blood sugar tester, and general purpose health management devices used for health management, such as a pulse measuring device and a scale. Generally, health management devices are not suitable for performing complicated tasks other than their intended function for displaying results of measurements. Furthermore, since sizes of displays or displayable colors are limited in such health management devices, it is often difficult to display various statistic values or developments of measured results. Therefore, it is convenient to transmit data including measured results from a health management device to a separate client device and process the data at the client device.

For example, in case of a patch-type thermometer, body temperature of a child may be periodically measured at a designated time interval and transmitted to a client device of a parent, and displayed at the client device, and thus the parent may check body temperature of the child without bothering the sick or sleeping child.

Furthermore, the client device 200 refers to a terminal capable of executing applications, such as a mobile phone, a smart phone, a personal digital assistant (PDA), a PDA phone, a laptop, and a smart TV, and may include all types of terminals that may be connected to the health management device recommending server 100 via a network.

The most popular client device may be a smart phone. A smart phone includes not only functions of a mobile phone, but also various other functions, thus being suitable for performing functions of a PDA or a small PC. Furthermore, a user may user a health management device in connection with a smart phone, thereby conveniently utilizing various smart phone-related functions in the health management device.

Data regarding health condition of a user measured by a health management device is transmitted from the health management device to a smart phone, so that the user may use a health management application designed for the smart phone without inputting data regarding health condition, thus being more convenient than a case of using the health management device alone.

Communication methods for connecting a client device to a health management device may include at least one from among Bluetooth communication, Zigbee communication, Wi-Fi communication, near field communication (NFC), ultra wide band (UWB) communication, ANT+ communication, infrared data association (IrDA) communication, and universal serial bus (USB) communication. Here, the USB communication includes not only wired USB communication, but also wireless USB communication. However, an exemplary embodiment is not limited to the above-stated communication methods.

The Bluetooth communication is one of the most popular close-distance communication methods using frequency hopping spread spectrum (FHSS) mechanism, where the Bluetooth special interest group (SIG), which is the body that oversees the development of Bluetooth standards, defines a separate technical specification for health management devices.

The Zigbee communication is a close-distance communication mainly used in vertical application fields, such as a sensor network, and complies with the 802.15.4 standard that defines low-power wireless personal area network (PAN).

The Wi-Fi communication is a close-distance communication for connecting to a network via an access point (AP), is considered as the synonym of wireless LAN, and complies with the 802.11b standard.

The NFC communication is one of radio frequency identification (RFID) techniques and is a noncontacting communication technique. Due to short communication range, NFC features excellent security and it is not necessary to set individual devices.

The UWB communication is a technique for dispersing signals throughout frequency bands up to several GHz by using ultra-short wave pulses. Since the UWB communication uses direct spectrum spread spectrum (DSSS) mechanism and pulses feature short cycles, it is easy to guarantee quality of service (QoS) and a plurality of paths causes high resistance to signal interference.

The ANT+ communication is mainly used in health management devices and is used to periodically transmit and monitor sensor data.

The IrDA communication is a communication method by which electronic devices may exchange data using infrared rays. Unlike a communication using radio waves, the IrDA communication is very stable, because no radio wave interference or malfunction of other devices occurs. However, due to linearity of an infrared ray, it is necessary to secure a particular angle and a line of sight (LOS).

The USB communication has been developed to standardize ports for connecting a personal computer (PC) and peripheral devices. Currently, the USB communication is widely used not only for connecting a PC and peripheral devices, but also for interconnecting terminals. Small-size terminals uses a mini USB or a micro USB, and a wireless USB utilizing the UWB technique is also available.

A communication protocol via which a client device and a health management device may connect to each other refers to standardized regulations for communication between terminals or devices and may vary according to communication methods or fields of applications. A profile via which a client device and a health management device may connect to each other refers to rules regarding a communication regulations to be used by an application for securing mutual connectivity between terminals or devices and may vary according to communication methods, communication protocols, and functions provided by the application.

The health information providing server 300 transmits a user's health information including health condition of the user or information regarding the user's fields of interest related to health management to the health management device recommending server 100. Although FIG. 1 shows that the health information providing server 300 is a device arranged separately from the client device 200, the health information providing server 300 may be integrated in the client device 200.

The health management device recommending server 100 determines a recommended health management device based the connectivity information regarding the client device 200 transmitted from the client device 200 and the user's health information transmitted from the health information providing server 300 and transmits information regarding the recommended health management device to the client device 200.

Therefore, the user may check the recommended health management device, which may be connected to his/her client device and is appropriate for his/her health condition or fields of interest related to heath management, via a display of the client device 200. Furthermore, the user may check detailed information regarding the recommended health management device displayed on the display via a user input and may easily decide whether to purchase the recommended health management device.

The network 600 interconnects the health management device recommending server 100, the client device 200, and the health information providing server 300. The network 600 includes a dedicated line, a LAN, a VAN, an intranet, a private telephone network, a public telephone network, a PSTN network, and combinations thereof, and includes comprehensive data communication networks for smooth communication between the respective network components shown in FIG. 1, such as a wired internet network, a wireless internet network, and a mobile wireless internet network.

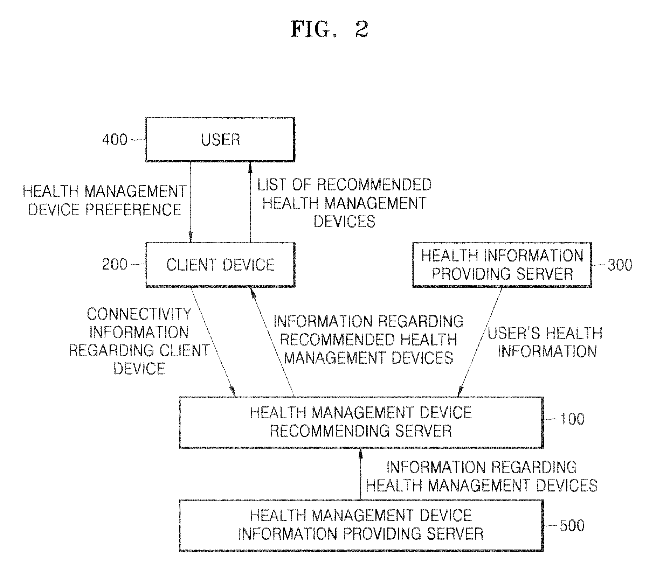

Hereinafter, a health management device recommending system according to an exemplary embodiment will be described in detail with reference to FIG. 2.

FIG. 2 is a block diagram showing a health management device recommending system using a user's health information and connectivity information regarding a client device according to an exemplary embodiment.

When a user 400 wants to check information regarding a health management device or to purchase a health management device, the user 400 connects the client device 200 to the health management device recommending server 100 by using a particular application installed on the client device 200 or an internet browser application installed on the client device 200.

When the client device 200 is connected to the health management device recommending server, the client device 200 transmits connectivity information regarding the client device to the health management device recommending server 100. The connectivity information regarding the client device refers to all information that includes information regarding communication methods, communication protocols, and profiles supported by the client device, i.e., refers to all information for connecting a health management device and a client device to each other.

The health information providing server 300 transmits a user's health information to the health management device recommending server 100. The user's health information may include not only information regarding medical history of the user, but also information regarding preferred sport activities or preferred diet of the user for health management.

Like FIG. 1, FIG. 2 also shows that the health information providing server 300 is a device arranged separately from the client device 200. However, as described above, the health information providing server may be integrated in the client device 200. If the health information providing server is integrated in the client device 200, the client device 200 may further include a storage unit for storing users' health conditions.

A health management device information providing server 500 stores information regarding health management devices, and, when the health management device recommending server 100 requests, transmits the information regarding health management devices to the health management device recommending server. The information regarding health management devices may include information regarding means of communication for health management devices to communicate with other devices, size, weight, measuring method, price, and retail store. Here, the other devices that may be connected to the health management device may be the client device 200.

The health management device recommending server 100 determine recommended health management devices based on the connectivity information regarding a client device received from the client device 200, the health information received from the health information providing server 300, and the information regarding health management devices received from the health management device information providing server 500 and transmits information regarding the recommended health management devices to the client device 200.

The client device 200 generates a list of recommended health management devices by using the information regarding recommended health management devices received from the health management device recommending server 100 and displays the generates list of the recommended health management devices on a display, so that the user 400 may check the list.

The user 400 may check information regarding a desired health management device from among health management devices included in the list of recommended health management devices and may easily determine whether to purchase the desired health management device. Furthermore, since the list of recommend health management devices include only health management devices that may be connected to the client device 200, inconvenience of purchasing a wrong health management device that may not be connected to the client device 200 may be reduced.

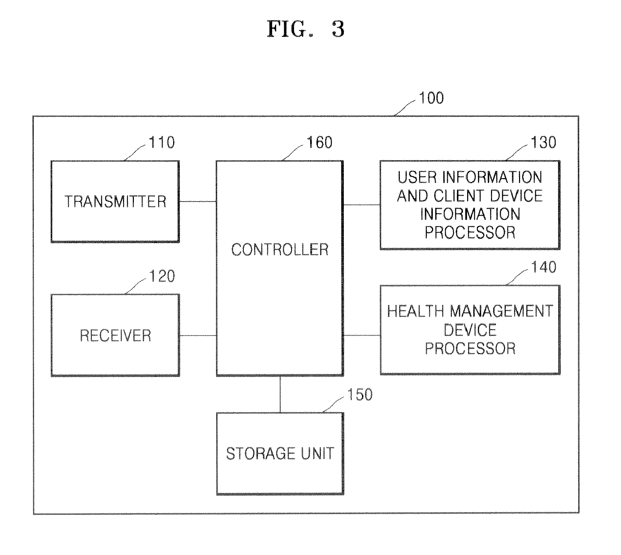

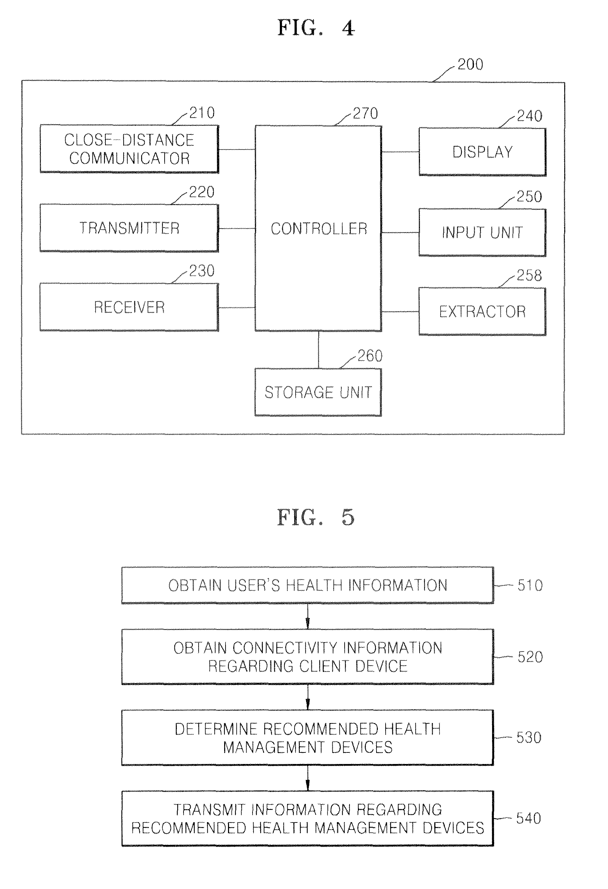

Hereinafter, referring to FIGS. 3 and 4, configurations of the health management device recommending server 100 and the client device 200 according to an exemplary embodiment will be described.

FIG. 3 is a diagram showing the detailed configuration of the health management device recommending server 100 according to an exemplary embodiment.

As shown in FIG. 3, the health management device recommending server 100 includes a transmitter 110, a receiver 120, a user information and client device information processor 130, a health management device processor 140, a storage unit 150, and a controller 160.

The transmitter 110 transmits information regarding recommended health management devices transmitted from the health management device processor 160 to the client device 200. According to an exemplary embodiment, the transmitter 110 may transmit user information to the health information providing server 300.

The receiver 120 receives the connectivity information regarding the client device 200 transmitted from the client device 200 and user's health information transmitted from the health information providing server 300. Furthermore, according to an exemplary embodiment; the receiver 120 may receive information regarding health management devices transmitted from the health management device information providing server 500.

The user information and client device information processor 130 processes user information transmitted from the client device 200 and transmits a health information requesting signal to be transmitted to the health information providing server 300 to the transmitter 110. The user information and client device information processor 130 may also process information regarding a client device transmitted from the client device 200, extract type, model name, and connectivity information regarding the client device, and transmit the extracted type, model name, and connectivity information regarding the client device to the health management device processor 140.

According to an exemplary embodiment; if user's health information is stored in a client device, the user information and client device information processor 130 may process user information transmitted from the client device and directly extract the user's health information. According to an exemplary embodiment, the user information and client device information processor 130 may process user information transmitted from a client device, further extract information regarding preference or age of a corresponding user, and transmit the extracted information to the health management device processor 140.

The health management device processor 140 determines recommended health management devices based on health information transmitted from the health information providing server 300 or the client device 200 and information regarding a client device transmitted from the client device 200. Furthermore, according to an exemplary embodiment, information regarding preference or age of a user may be further utilized for determining recommended health management devices.

The health management device processor 140 selects health management devices a user needs by using the user's health information and determines recommended health management devices that may be connected to the client device 200 from among the selected health management devices by using information regarding a client device. If additional information, such as information regarding preference or age of a user, is used to recommend health management devices, health management devices that are more suitable for the corresponding user may be recommended.

The storage unit 150 stores various information, such that the health management device recommending server 100 may determine recommended health management devices based on connectivity information regarding the client device 200 transmitted from the client device 200 and user's health information transmitted from the health information providing server 300 and display recommended health management devices. Furthermore, the storage unit 150 may store information regarding recommended health management devices for respective users.

The controller 160 controls the overall operation of the health management device recommending server 100 and controls the transmitter 110, the receiver 120, the user information and client device information processor 130, the health management device processor 140, and the storage unit 150, such that the health management device recommending server 100 determines recommended health management devices based on connectivity information regarding the client device 200 transmitted from the client device 200 and user's health information transmitted from the health information providing server 300.

FIG. 4 is a diagram showing the detailed configuration of the client device 200 according to an exemplary embodiment.

As shown in FIG. 4, the client device 200 includes a close distance communicator 210, a transmitter 220, a receiver 230, a display 240, an input unit 250, a storage unit 260, and a controller 260.

The close distance communicator 210 performs a close-distance communication between the client device 200 and a health management device, where the single client device 200 may support a plurality of close-distance communication methods. As described above, close distance communication methods via which a client device and a health management device may communicate with each other may include at least one from among Bluetooth communication, Zigbee communication, Wi-Fi communication, near field communication (NFC), ultra wide band (UWB) communication, ANT+ communication, infrared data association (IrDA) communication, and universal serial bus

(USB) communication. Here, the USB communication includes not only wired USB communication, but also wireless USB communication. However, the present exemplary embodiment is not limited to the above-stated communication methods.

Furthermore, although a close-distance communication is mainly used as a communication method for connecting a health management device and a client device to each other, the present exemplary embodiment is not limited thereto. If a communication method other than close-distance communication method is used, the close distance communicator 210 may be replaced with a different component suitable for the corresponding communication method.

The transmitter 220 transmits user information and information regarding a client device to the health management device recommending server 100. According to an exemplary embodiment, the transmitter 220 may also transmit user information and the user's health information together. Furthermore, according to an exemplary embodiment, if a user wants to purchase a particular health management device from among recommended health management devices, the transmitter 220 may transmit a purchase requesting signal or a seller information.

The receiver 230 receives information regarding recommended health management devices from the health management device recommending server 100. Furthermore, according to an exemplary embodiment, if a user wants to purchase a particular health management device from among recommended health management devices, the receiver 230 may receive purchase information or payment information regarding the desired health management device.

The display 240 displays a list of recommended health management devices generated by using information regarding recommended health management devices received by the receiver 230 on a display, so that a user may check the list of recommended health management devices.

The input unit 250 receives user preference information regarding health management devices or a user input for checking detailed information regarding a particular health management device from among recommended health management devices. Furthermore, in case of a touch-type client device, the input unit 250 may be integrated in a display.

According to an exemplary embodiment, if user's health information is stored in the client device 200, the user may directly input health information to the client device 200 via the input unit 250.

The storage unit 260 stores user information and information regarding a client device and various pieces of information related to information regarding recommended health management devices transmitted from the health management device recommending server 100. According to an exemplary embodiment, if user's health information is stored in a client device, the storage unit 260 may store the information regarding a client device and may additionally store user preference information regarding health management devices or health management device search information.

The controller 270 controls the overall operation of the client device 200 and controls the close distance communicator 210, the transmitter 220, the receiver 230, the display 240, the input unit 250, and the storage unit 260, such that user information and information regarding a client device may be transmitted to the health management device recommending server 100 and a list of recommended health management devices may be provided to a user based on information regarding recommended health management devices received from the health management device recommending server 100.

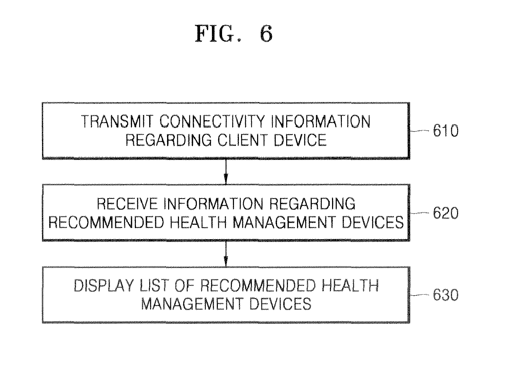

Hereinafter, operations of the health management device recommending server 100 and the client device 200 according to an exemplary embodiment will be described with reference to FIGS. 5 and 6.

FIG. 5 is a flowchart showing operations of the health management device recommending server 100 according to an exemplary embodiment.

The health management device recommending server 100 obtains user's health information to recommend health management devices suitable for a user (operation 510). The user's health information includes information regarding medical history of the user and information regarding health management of the user, such as information regarding preferred sport activities and preferred diet of the user for health management.

The health management device recommending server 100 may provide user information received from the client device 200 to the health information providing server 300 and may receive the user's health information from the health information providing server 300. Furthermore, the health management device recommending server 100 may receive the user's health information directly from the client device 200.

The health management device recommending server obtains connectivity information regarding a client device transmitted from the client device (operation 520). The connectivity information regarding a client device is information regarding communication methods via which the client device and a health management device may be connected to each other and may include information regarding close-distance communication methods and information regarding communication protocols and profiles to be connected to health management devices.

As described above, close distance communication methods via which a client device and a health management device may communicate with each other may include at least one from among Bluetooth communication, Zigbee communication, Wi-Fi communication, near field communication (NFC), ultra wide band (UWB) communication, ANT+ communication, infrared data association (IrDA) communication, and universal serial bus (USB) communication. Here, the USB communication includes not only wired USB communication, but also wireless USB communication. However, the present exemplary embodiment is not limited to the above-stated communication methods.

The health management device recommending server determines recommended health management devices suitable for a user by using the obtained connectivity information regarding a client device and the user's health information (operation 530) and transmits information regarding the recommended health management devices to the client device (operation 540). The information regarding recommended health management devices may include information regarding device names, model names, communication modules integrated in the health management devices, sizes, weights, and other additional information. The information regarding recommended health management devices may further includes purchase-related information, such as prices and seller information.

FIG. 6 is a flowchart showing a method that a client device requests a health management device recommending server to recommend health management devices and receives information regarding recommended health management devices from the health management device recommending server, according to an exemplary embodiment.

If the user 400 requests information regarding health management devices, the client device 200 transmits connectivity information regarding the client device 200 to the health management device recommending server 100. The connectivity information regarding the client device 200 refers to information regarding communication methods via which the client device 200 and a health management device may be connected to each other and may include information regarding close-distance communication methods and information regarding communication protocols and profiles to be connected to health management devices.

As described above, close distance communication methods via which a client device and a health management device may communicate with each other may include at least one from among Bluetooth communication, Zigbee communication, Wi-Fi communication, near field communication (NFC), ultra wide band (UWB) communication, ANT+ communication, infrared data association (IrDA) communication, and universal serial bus (USB) communication. Here, the USB communication includes not only wired USB communication, but also wireless USB communication. However, the present exemplary embodiment is not limited to the above-stated communication methods.

Here, the client device 200 may transmit user's health information to the health management device recommending server 100 together with the connectivity information regarding the client device 200. The health management device recommending server 100 determines recommended health management devices by using the connectivity information regarding the client device and the user's health information and transmits the information regarding recommended health management devices to the client device 200. The information regarding recommended health management devices may include information regarding device names, model names, communication modules integrated in the health management devices, sizes, weights, and other additional information. According to an exemplary embodiment, the information regarding recommended health management devices may further includes purchase-related information, such as prices and seller information.

The client device 200 generates a list of recommended health management devices by using the received information regarding recommended health management devices and displays the generated list, so that a user may check the list. According to an exemplary embodiment, if the information regarding recommended health management devices includes information regarding purchase of health management devices, the client device 200 may also display information regarding sellers of health management devices in a screen image for displaying the information regarding recommended health management devices, where hyperlinked buttons may be used for a user to easily move to seller websites.

A user may check the list of recommended health management devices via the client device 200 and may obtain detailed information regarding desired health management devices from among the health management devices included in the list. According to an exemplary embodiment, the user may also check information regarding prices and sellers and may easily move to seller websites via hyperlinked buttons, and thus the user may easily decide to purchase health management devices and make payment.

According to an exemplary embodiment, if software for checking recommended health management devices of a client device has payment function, the user may purchase a health management device and make payment therefor at the stage of checking information regarding health management devices without moving to seller websites.

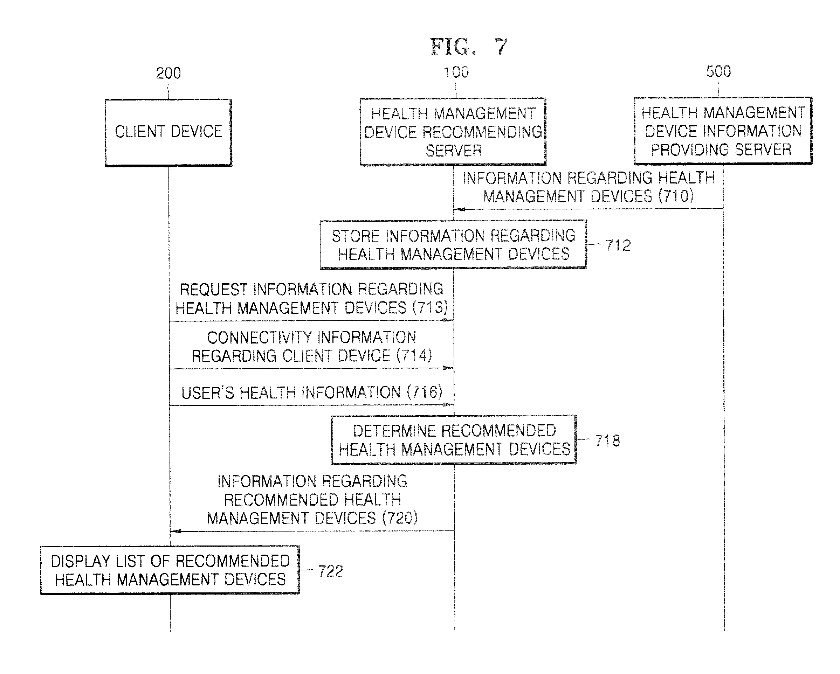

Hereinafter, operations of the health management device recommending server 100 and the client device 200 according to an exemplary embodiment will be described with reference to FIGS. 7 through 10.

FIG. 7 is a detailed flowchart showing a method of recommending health management devices by using user's health information and connectivity information regarding a client device according to an exemplary embodiment.

FIG. 7 is a detailed flowchart showing a method of recommending health management devices by using user's health information and connectivity information regarding a client device stored in the client device. Examples of servers for storing information regarding health management devices and managing information regarding health management devices include product information server operated by manufacturers of health management devices, which will be referred to as the health management device information providing servers 500.

Although FIG. 7 shows the only one health management device information providing server 500 for convenience of explanation, a plurality of health management device information providing servers may exist in correspondence to respective manufacturers. According to an exemplary embodiment, the health management device information providing server 500 does not store and manage information regarding health management devices of only a particular manufacture, but may store and manage information regarding health management devices of a plurality of manufacturers.

Since information regarding health management devices is necessary for the health management device recommending server 100 to recommend health management devices suitable for a user, information regarding health management devices stored in the health management device recommending server 100 may be extracted or information regarding health management devices may be received from the health management device information providing server 500.

Therefore, in case of receiving information regarding health management devices from the health management device information providing server 500, the health management device recommending server 100 may need an operation 710 for receiving information regarding health management devices from the health management device information providing server 500 and an operation 712 for storing the received information regarding health management devices.

The client device 200 transmits a signal for requesting information regarding recommended health management devices to the health management device recommending server 100 (operation 713) and transmits connectivity information regarding a client device and user's health information, which are necessary for determining recommended health management devices, to the health management device recommending server 100 (operations 714 and 716).

The health management device recommending server 100 determine recommended health management devices based on the received connectivity information regarding a client device and the user's health information (operation 718) and transmit information regarding the recommended health management devices to the client device 200 (operation 720). The client device 200 displays a list of recommend health management devices on a display based on the received information regarding recommended health management devices.

The health management device recommending server 100 selects health management devices a user needs particularly by using information regarding medical history and fields of interest and determines health management devices that may be connected to the client device 200 from among the selected health management devices by using the information regarding a client device. In case of using information regarding a user's preference or age to recommend health management devices, health management devices more suitable for the user may be recommended.

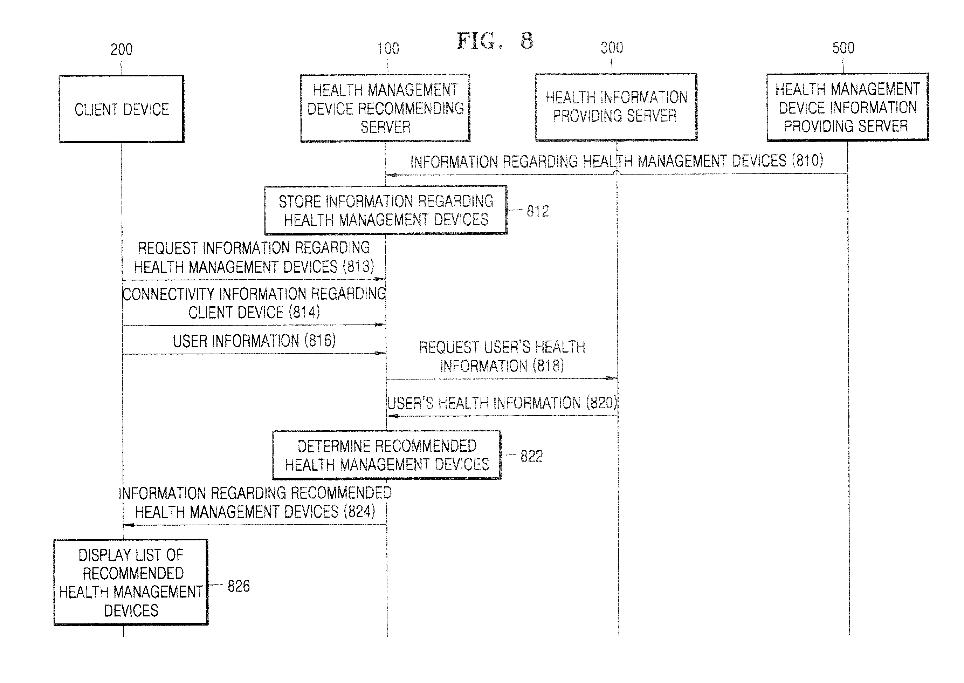

FIG. 8 is a detailed flowchart showing a method of recommending health management devices using user's health information and connectivity information regarding a client device according to an exemplary embodiment.

FIG. 8 is a detailed flowchart showing a method of recommending health management devices using connectivity information regarding a client device and user's health information stored in a separate health information providing server. An operation 810 in which the health management device recommending server 100 receives information regarding health management devices from the health management device information providing server 500 and an operation 812 in which the health management device recommending server 100 stores the information regarding health management devices are identical to the operations according to an exemplary embodiment shown in FIG. 7. However, unlike an exemplary embodiment shown in FIG. 7, the user's health information is not stored in a client device, and thus operations thereafter are slightly different from those of an exemplary embodiment shown in FIG. 7.

The client device 200 transmits a signal for requesting information regarding recommended health management device to the health management device recommending server 100 (operation 813) and transmits connectivity information regarding a client device and user information for identifying a user to the health management device recommending server 100 (operations 814 and 816). The health management device recommending server requests user's health information to the health information providing server 300 to obtain health information regarding a user of a terminal which requested the information regarding recommended health management devices (operation 818), and the health information providing server 300, which received a signal for requesting health information transmits the corresponding user's health information to the health management device recommending server (operation 820).

Operations thereafter are identical to those of an exemplary embodiment shown in FIG. 7. The health management device recommending server 100 determine recommended health management devices based on the received connectivity information regarding a client device and the user's health information (operation 822) and transmits information regarding the recommended health management devices to the client device (operation 824). The client device 200 displays a list of recommend health management devices on a display based on the received information regarding recommended health management devices (operation 826).

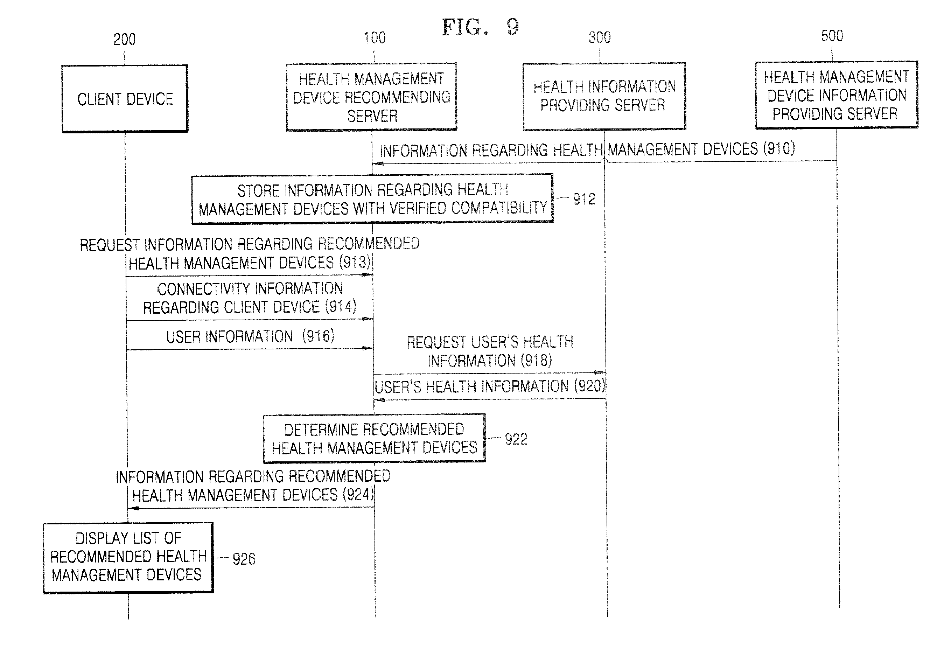

FIG. 9 is a detailed flowchart of a method of recommending health management devices by using user's health information and connectivity information regarding the client device, according to an exemplary embodiment.

Like FIG. 8, FIG. 9 is also a detailed flowchart showing a method of recommending health management devices using connectivity information regarding a client device and user's health information stored in a separate health information providing server. However, the method shown in FIG. 9 differs from the method shown in FIG. 8 for storing information regarding health management devices in the health management device information providing server 500.

In exemplary embodiments shown in FIGS. 7 and 8, the health management device recommending server 100 receives information regarding health management devices transmitted from the health management device information providing server 500 and stores information regarding all health management devices. However, in an exemplary embodiment shown in FIG. 9, information regarding only health management devices with verified compatibility from among health management devices corresponding to the received information is stored.

The verification of compatibility is necessary to guarantee terminal connectivity, which is an important factor for recommending health management devices. To this end, based on results of connectivity tests between a client device and health management devices, information regarding a health management device is not stored in case where the client device and the health management device are not connected to each other even by using a same communication method or connection success rate is below or equal to a designated level.

By performing such the compatibility verification and storing information regarding verified health management devices (operation 912), health management devices without guaranteed connectivity may be basically excluded, and thus more reliable recommendation of health management devices may be made. Furthermore, in the operation 912 for verifying compatibility and storing information regarding verified health management devices, when information regarding health management devices is received, a device model may be checked and it may be determined whether to store information based on results of previous tests stored in the health management device recommending server in advance or based on results of compatibility tests transmitted together with the information regarding health management devices.

Operations after information regarding health management devices with verified compatibility is stored are identical to those shown in FIG. 8.

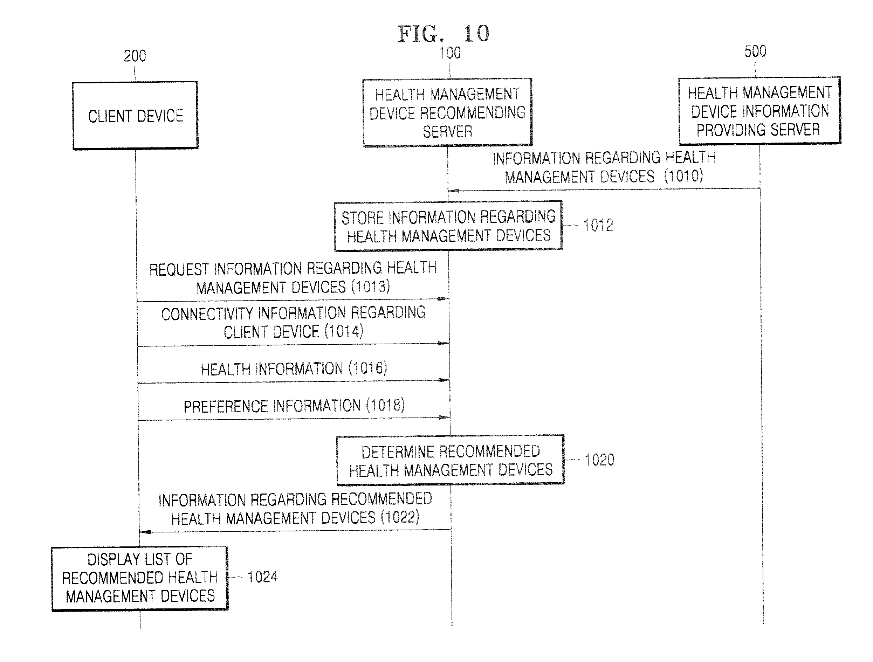

FIG. 10 is a detailed flowchart showing a method of recommending health management devices by using user's health information and connectivity information regarding the client device, according to an exemplary embodiment.

Like FIG. 7, FIG. 10 is a detailed flowchart showing a method of recommending health management devices by using user's health information and connectivity information regarding the client device that are stored in the client device. However, in an exemplary embodiment shown in FIG. 10, the client device 200 further transmits user preference information to determine recommend health management devices.

The user preference information is information regarding a user's preference regarding health management devices, where a user's preference may be changed based on locations or purposes the user plans to use health management devices. For example, if a user wants a device for checking heart rate during a sport activity, preference may be higher for smaller and lighter devices, and a user may wear the device at different locations according to the user's preference, e.g., an arm-band type or a wrist-watch type.

On the contrary, in case of a household health management device, a user may prefer a large or heavy health management device, which has memory functions for continuous health management. Furthermore, in case of a health management device to be used at a hospital, precision and durability of a health management device may be more important factor regarding preference than size or weight. Furthermore, the user preference information may include information regarding a user's desired price range.

As described above, by determining recommended health management devices by further using user preference information, a user may be prevented from being provided unnecessary information, and thus the user may easily check information regarding recommended health management devices.

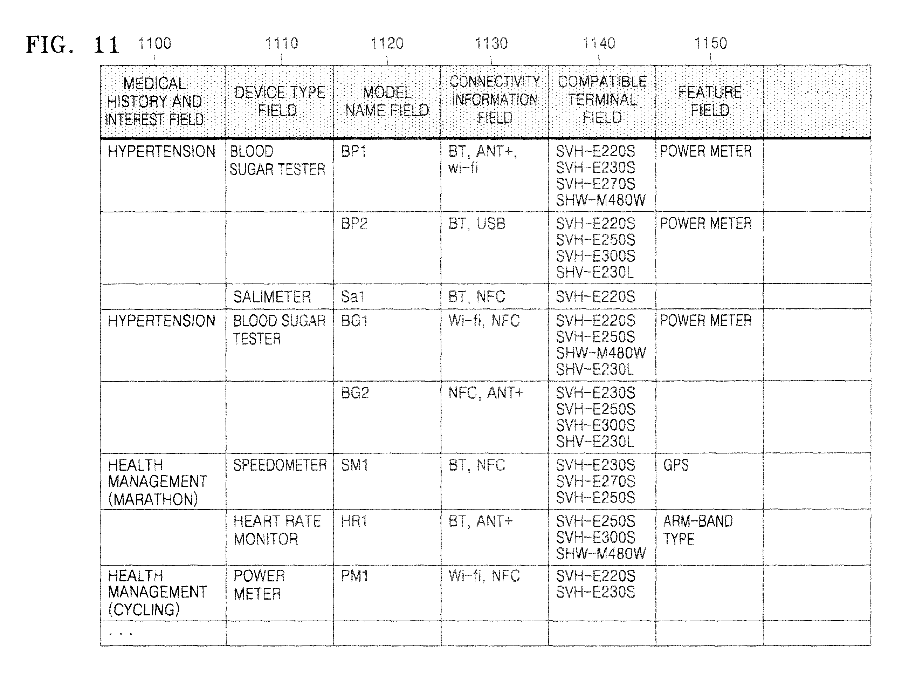

Hereinafter, referring to FIG. 11, an example of tables including information regarding health management devices according to an exemplary embodiment will be described.

FIG. 11 is a diagram showing an example of tables including information regarding health management devices according to an exemplary embodiment.

As shown in FIG. 11, the table including information regarding health management devices according to an exemplary embodiment may include a medical history and interest field 1100, a device type field 1110, a model name field 1120, a connectivity information field 1130, a compatible terminal field 1140, and a feature field 1150.

The medical history and interest field 1100 includes information regarding medical history or fields of interest of a user necessary for categorizing health management devices. For example, the information may include information regarding chronic diseases requiring continuous management, such as hypertension or diabetes, or the user's daily sport activities, such as marathon, cycling, or swimming.

The device type field 1110 includes information regarding type of a health management device related to the medical history and interest field 1100. For example, a hypertension patient needs a blood pressure gauge for blood pressure management and may likely need a salimeter for a dietary treatment. Meanwhile, a diabetes patient may need a blood sugar tester, and, since a diabetes patient often has hypertension as a complication, information regarding a blood sugar tester and information regarding a blood pressure gauge may be recorded together if an occasion demands.

Furthermore, if the user frequently runs marathon for health management, the user may likely want to purchase a speedometer and a heart rate monitor. Therefore, if the medical history and interest field 1100 indicates marathon, information regarding a speedometer and a heart rate monitor may be recorded to the device type field 1110. If a user frequently goes cycling, information regarding a power meter and a speedometer may be recorded to the device type field 1110.

The model name field 1120 corresponds to the device type field 1110 and includes information regarding name of a corresponding health management devices.

The connectivity information field 1130 includes connectivity information, which is information regarding communication methods via which corresponding health management devices corresponding to the model name of the model name field 1120 may be connected to a client device. Like the connectivity information regarding a client device, the connectivity information regarding a corresponding health management device is information regarding communication methods via which a client device and the corresponding health management device may be connected to each other and may include information regarding communication methods, communication protocols, and communication profiles supported by the health management device.

The compatible terminal field 1140 includes information regarding model names of client devices compatible with corresponding model names. Here, the compatibility may be determined based on communication methods, communication protocols, and communication profiles to be connected to client devices included in the connectivity information or results of other compatibility tests.

The feature field 1150 includes information regarding features of the corresponding health management device, such as information regarding size or weight of the corresponding health management device and information indicating whether the corresponding health management device is a portable type or a stationary type. Furthermore, in case of a health management device to be used during a sport activity, such as a speedometer or a heart rate monitor, the feature field 1150 may further include information indicating whether GPS function is included or information indicating whether a MP3 playback function is included.

Furthermore, although not shown in FIG. 11, the table including information regarding health management devices may further include a size field, a weight field, and purchase information field including price information.

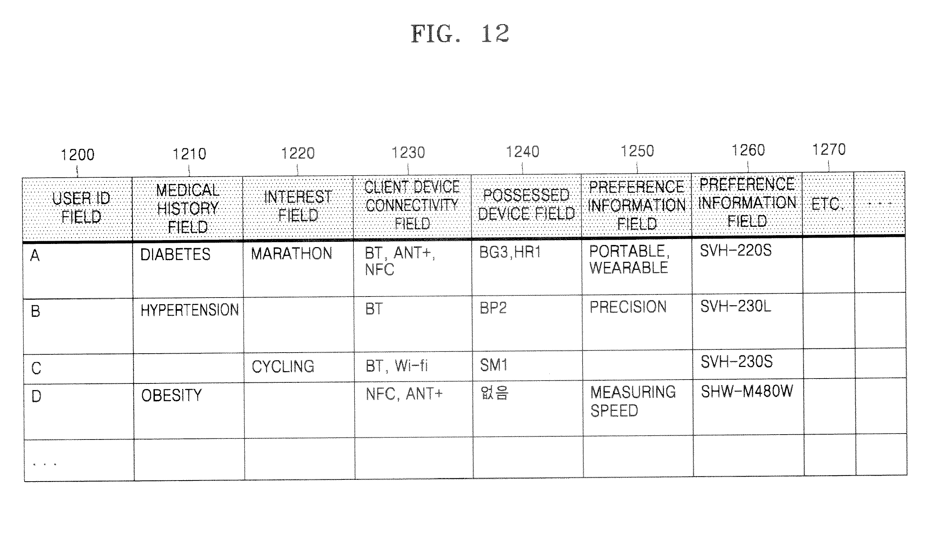

Hereinafter, an example of tables including users' health information will be described with reference to FIG. 12.

FIG. 12 is a diagram showing an example of tables including users' health information according to an exemplary embodiment.

As shown in FIG. 12, the table including users' health information according to an exemplary embodiment includes a user ID field 1200, a medical history field 1210, an interest field 1220, a client device connectivity field 1230, a possessed device field 1240, a preference information field 1250, a client device model name field 1260, and a miscellaneous field 1270.

The user ID field 1200 includes information for identifying a user. By continuously managing and updating a medical history or a history of purchasing health management devices by using the user ID field 1200, more information for recommending health management devices suitable for a corresponding user may be secured, and thus more accurate recommendation may be made.

The medical history field 1210 includes information regarding a medical history of a user corresponding to a user ID, whereas the interest field 1220 includes information regarding fields of interest related to health management, such as information regarding sport activities continuously performed by a user corresponding to a user ID.

The client device connectivity field 1230 includes connectivity information, which includes information regarding communication methods for a client device corresponding to a user ID to be connected to health management devices. The connectivity information regarding a client device may include information regarding close-distance communication methods and information regarding communication methods, communication protocols, and communication profiles supported by the health management device.

The possessed device field 1240 includes information regarding health management devices already possessed by a user corresponding to a user ID. When recommended health management devices are determined, overlapping devices may be excluded from recommended health management devices by using the possessed device field 1240. According to an exemplary embodiment, user preference information may be obtained by analyzing features of possessed devices.

The preference information field 1250 includes information regarding preference of a user corresponding to a user ID with respect to health management devices. For example, a user with frequent outdoor activities may prefer portable or wearable health management devices, where measurement precision may be an important factor for a particular user to decide to purchase health management devices, and measurement speed may be an important factor for another user to decide to purchase health management devices. Such information is included in the preference information.

The client device model name field 1260 includes information regarding model name of a client device. If connectivity information regarding the client device is lost or not recorded, the connectivity information regarding the client device may be restored or recorded by using the corresponding model name.

The miscellaneous field 1270 may include various other information, such as age or gender of a user. According to an exemplary embodiment, information regarding preference of the user may be obtained or a sequence of recommending health management devices may be determined based on statistics of preferences of users of corresponding gender and ages regarding health management devices by using information regarding age and gender of the user.

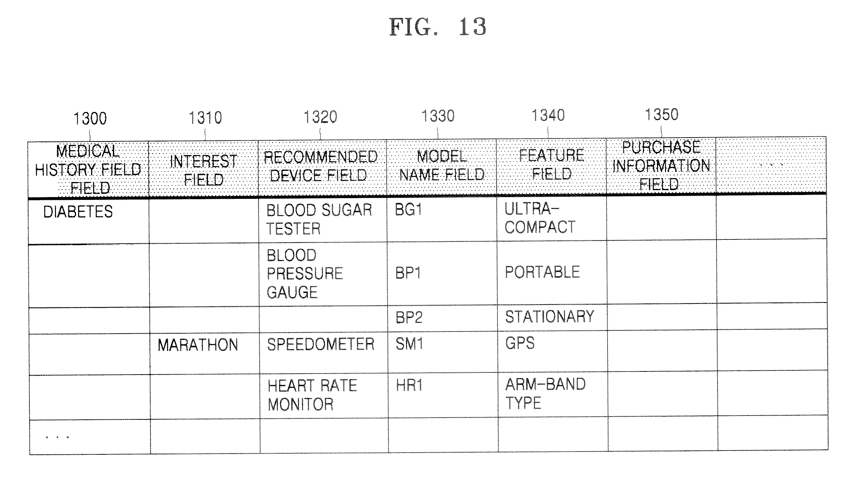

Hereinafter, referring to FIG. 13, an example of results of recommending health management devices by using user's health information and connectivity information regarding a client device, according to an exemplary embodiment.

FIG. 13 is a diagram showing an example of screen images showing results of recommending health management devices by using user's health information and connectivity information regarding a client device, according to an exemplary embodiment. FIG. 13 shows a screen image showing a result of recommending health management devices with respect to a user A by using the table including information regarding health management devices shown in FIG. 11 and the table including users' health information shown in FIG. 12.

As shown in FIG. 13, the screen image showing a result of recommending health management devices using user's health information and connectivity information regarding a client device includes a medical history field 1300, an interest field 1310, a recommended device field 1320, a model name field 1330, a feature field 1340, and a purchase information field 1350.

Referring to the table including users' health information shown in FIG. 12, the medical history field 1210 indicates diabetes and the interest field 1220 indicates marathon with respect to a user A. Referring to the table including information regarding health management devices shown in FIG. 11, types of recommended devices include a blood sugar tester in case where the medical history and interest field 1100 indicates diabetes, and, since a diabetes patient often has hypertension as a complication, a blood pressure gauge may be recommended together as described above. Furthermore, if the medical history and interest field indicates marathon, types of recommended devices include a speedometer and a heart rate monitor.

Since the user A has diabetes and is interested in marathon, the medical history field 1300 indicates diabetes and the interest field 1310 indicates marathon. In correspondence thereto, the recommended device field 1320 indicates a blood sugar tester, a blood pressure gauge, a speedometer, and a heart rate monitor.

Referring back to the table including users' health information shown in FIG. 12, the client device connectivity field 1230 regarding the user A indicates BT, ANT+, and NFC, and thus models supporting the corresponding communication methods are searched for from among related health management devices. According to an exemplary embodiment, models already possessed by the user A may be excluded by using the possessed device field 1240.

Furthermore, referring to the preference information field 1250 of the table including users' health information, the user A prefers portable or wearable health management device. Therefore, only devices corresponding to the preference information may be included in a search result or devices corresponding to the preference information may be displayed at the top of a list of recommended health management devices.

According to the above embodiments, the health management device recommending server 100 recommends health management devices. However, as the client device 200 has more functions, the recommendation of health management device may be performed by the client device 200.

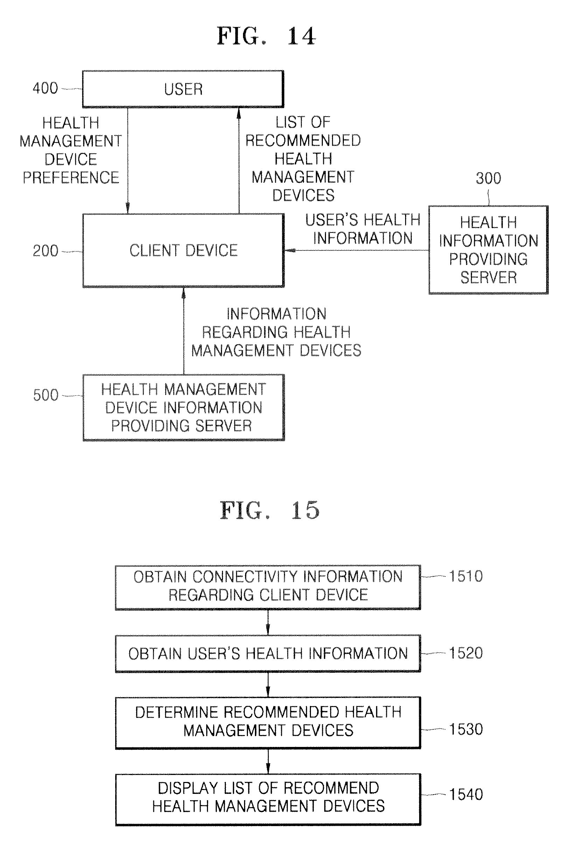

Referring to FIGS. 14 and 15, a system for recommending health management devices using a client device according to an exemplary embodiment will be described in detail.

FIG. 14 is a block diagram showing a health management device recommending system using connectivity information regarding a client device, according to an exemplary embodiment.

When the user 400 checks information regarding health management devices or wants to purchase health management devices, the user 400 executes a particular application installed on the client device 200 possessed by the user 400, e.g., a health management device recommending application.

When the health management device recommending application is executed on the client device 200, the client device 200 extracts connectivity information regarding communication methods integrated in the client device 200 or connectivity information regarding communication methods that may be performed by using an external module. The client device 200 determines recommend health management devices that may be connected to the client device 200, based on connectivity information regarding the client device 200 related to extracted communication methods and information regarding health management devices.

The connectivity information regarding the client device 200 executed on the client device 200 is an application program that is or may be installed on the 200 and may either independently determine recommended health management devices via the client device 200 or determine recommended health management devices together with the health management device recommending server 100, the health information providing server 300, or the health management device information providing server 500.

Here, the information regarding health management devices for determining recommended health management device may be stored in the storage unit 260 of the client device 200. According to an exemplary embodiment, the information regarding health management devices for determining recommended health management device may be stored in the health management device information providing server 500 which manages information regarding health management devices. If the information regarding health management devices is stored in the health management device information providing server 500, the client device 200 requests information regarding health management devices to the health management device information providing server 500.

Even if information regarding health management devices is stored in the storage unit 260 of the client device 200, to update information regarding health management devices, the client device 200 may request information regarding health management devices to the health management device information providing server 500 which manages information regarding health management devices, receives the information regarding health management devices, and manage the information regarding health management devices stored in the storage unit 260.

The health management device information providing server 500 may be a server operated by the manufacturer of a health management device or may be a dedicated server for managing information regarding health management devices of a plurality of manufacturers.

According to an exemplary embodiment, the client device 200 may further use user's health information for recommending more suitable health management devices to a user. As described above, the user's health information may include information regarding health status of a user or information regarding fields of interest related to health management.

The user's health information may be stored in the storage unit 260 of the client device 200. The client device 200 may include an extractor 258 which extracts user's health information from the storage unit 260 and uses the extracted user's health information for determining recommended health management devices that may be connected to the client device 200.

The user's health information may be stored in the health information providing server 300 separate from the client device 200. The client device may transmit a health condition requesting signal to the health information providing server 300 via the transmitter 220 and receives health information transmitted form the health information providing server 300 via the receiver 230. The client device 200 uses the received user's health information for determining recommended health management devices that may be connected to the client device 200.

According to an exemplary embodiment, to recommend health management devices that are more suitable for a user, the client device 200 may use additional information other than connectivity information regarding the client device 200, such as a user preference information regarding health management devices or information regarding age or gender of the user.

As described above, a user's preference information is information regarding preference of a user with respect to health management devices, where preference may be changed based on locations or purposes the user plans to use health management devices. For example, if a user wants a device for checking heart rate during a sport activity, preference may be higher for smaller and lighter devices, and a user may wear the device at different locations according to the user's preference, e.g., an arm-band type or a wrist-watch type.

On the contrary, in case of a household health management device, a user may prefer a large or heavy health management device, which has memory functions for continuous health management. Furthermore, in case of a health management device to be used at a hospital, precision and durability of a health management device may be more important factor regarding preference than size or weight. Furthermore, the user preference information may include information regarding a user's desired price range.

In case of using information regarding age and gender of a user, information regarding preference of the user may be obtained or a sequence of recommending health management devices may be determined based on statistics of preferences of users of corresponding gender and ages regarding health management devices by using information regarding age and gender of the user.

When recommended health management devices are determined, the client device 200 provides a list of recommended health management devices including model names of corresponding health management devices and information regarding the corresponding health management devices, so that a user may check the list.

Here, the list of recommend health management devices may be composed based on priorities, and health management devices having higher priority may be display first. The priority may be determined by combining user's health information, information regarding preference of a user, age, and gender, or may be determined based on a priority set by a user in advance.

FIG. 15 is a flowchart showing operations of the client device 200 according to an exemplary embodiment.

If the user 400 requests information regarding health management devices, the client device 200 obtains connectivity information regarding the client device 200 (operation 1510). The connectivity information regarding a client device refers to information regarding communication via which a client device and health management device may be connected to each other and may include information regarding communication methods integrated in the client device 200 or communications methods that may be used via an external module, communication protocols, and communication profiles.

As described above, close distance communication methods via which a client device and a health management device may communicate with each other may include at least one from among Bluetooth communication, Zigbee communication, Wi-Fi communication, near field communication (NFC), ultra wide band (UWB) communication, ANT+ communication, infrared data association (IrDA) communication, and universal serial bus (USB) communication. Here, the USB communication includes not only wired USB communication, but also wireless USB communication. However, the present exemplary embodiment is not limited to the above-stated communication methods.

According to an exemplary embodiment, the client device 200 obtains user's health information (operation 1520). The user's health information refers to information regarding medical history of the user and information regarding fields of interest related to health management of the user, such as information regarding preferred sport activities and preferred diet of the user for health management.

Since user's health information includes information regarding medical history of a user and information regarding fields of interest related to health management, health management devices that are more suitable for the corresponding user may be recommended by further using the user's health information. The user's health information may be stored in the storage unit 260 of the client device 200 or may be stored in the separate health information providing server 300 and may be transmitted to the client device 200 when the client device 200 requests the user's health information.

Although not shown, according to an exemplary embodiment, the client device 200 may determine recommended health management devices by further using preference information regarding health management devices and information regarding age and gender of a user.

The client device 200 determine recommended health management devices by using obtained connectivity information regarding the client device, user's health information, or preference information (operation 1530). When recommended health management device are determined, the client device 200 extracts information regarding recommended health management devices by using information regarding health management devices stored in the client device 200 or information regarding health management devices received from the health management device information providing server 500.

The information regarding recommended health management devices may include information regarding device names, model names, information regarding communication modules integrated in the health management devices, sizes, weights, and other information. The information regarding recommended health management devices may further include information related to purchase of health management devices, such as prices and sellers, other than the information stated above.

The client device 200 generates a list of recommended health management devices by using the received information regarding recommended health management devices and displays the generated list, so that the user may check the list.

Here, the list of recommend health management devices may be composed based on priorities, and health management devices having higher priority may be display first. The priority may be determined by combining user's health information, information regarding preference of a user, age, and gender, or may be determined based on a priority set by a user in advance.

According to an exemplary embodiment, if information regarding recommended health management devices includes information regarding purchase of health management devices, the client device 200 may display information regarding sellers in a screen image for checking information regarding recommended health management devices and the user may easily move to a corresponding seller's website via a hyperlinked button.

A user may check a list of recommended health management devices and may check detailed information regarding a desired health management device from among health management devices included in the list via the client device 200. According to an exemplary embodiment, if detailed information regarding a recommended health management device includes information regarding purchase of the corresponding health management device, the user may check information regarding price and sellers, may easily move to a seller website via a hyperlinked button, and may easily decide purchase of the corresponding health management devices and make payment therefor.

According to an exemplary embodiment, if a recommended health management device checking application of the client device 200 has a payment function, the user may purchase a health management device and make payment therefor at the stage of checking information regarding health management devices without moving to a seller websites.

As described above, according to the one or more of the above exemplary embodiments, health management devices that may be connected to a client device may be recommended.

Furthermore, as described above, according to one or more of exemplary embodiments, health management devices satisfying conditions demanded by a user may be recommended by using user's health information and preference information.

Exemplary embodiments may be implemented as computer instructions which can be executed by various computer means, and recorded on a computer-readable medium. The computer-readable medium may include program commands, data files, data structures or a combination thereof. Program instructions recorded on the medium may be particularly designed and structured for exemplary embodiments or available to those skilled in computer software. Examples of the computer-readable recording medium include magnetic media, such as a hard disk, a floppy disk, and a magnetic tape; optical media, such as a compact disk-read only memory (CD-ROM) and a digital versatile disc (DVD); magneto-optical media, such as floptical disks; a read-only memory (ROM); a random access memory (RAM); and a flash memory. The medium may be a transmission medium, such as an optical or metal line, a waveguide, or carrier waves transferring program commands, data structures, and the like. Program commands may include, for example, a high-level language code that can be executed by a computer using an interpreter, as well as a machine language code made by a complier. Hardware described herein may be embodied as one or more software modules to implement exemplary embodiments.

The foregoing exemplary embodiments and advantages are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A method implemented by a health management device recommending server which recommends health management device by using connectivity information of a client device, the method comprising:
receiving the connectivity information of the client device, from the client device;
recommending health management device, which is able to be connected to the client device, based on the received connectivity information and information about the health management device; and
transmitting information of the recommended health management device to the client device.

2. The method of claim 1, wherein the connectivity information comprises information of at least one of a communication method and a communication protocol supported by the client device.

3. The method of claim 1, wherein the receiving the connectivity information further comprises receiving user's health information from at least one of the client device and a health information providing server, and
the recommending the health management device further comprises recommending the health management device which relates to a health condition of the user, from the health management device that is able to be connected to the client device, based on the received user's health information.

4. The method of claim 3, wherein the receiving the connectivity information further comprises receiving information of a preference of the user about the health management device, and
the recommending the health management device further comprises recommending the health management device based on the preference information of the user.

5. The method of claim 4, wherein the preference information of the user comprises information of at least one of body parts to which the health management device is to be attached, a user's preference of brands of the health management device, and a history of purchasing the health management device.

6. A server that recommends health management device based on connectivity information of a client device, the server comprising:
a receiver, which receives the connectivity information of the client device, from the client device;
a controller, which recommends health management device, which is able to be connected to the client device, based on the received connectivity information and information about health management device; and
a transmitter, which transmits information about the recommended health management device to the client device.

7. A method of recommending health management device by using connectivity information of a client device, the method comprising:
extracting the connectivity information of the client device; and
recommending the health management device that is able to be connected to the client device based on the extracted connectivity information of the client device and information about the health management device.

8. The method of claim 7, further comprising extracting user's health information from the client device,
wherein the recommending the health management device comprises recommending those health management devices which relate to a health condition of the user, from the health management devices that are able to be connected to the client device, based on the extracted user's health information.

9. The method of claim 8, further comprising:
requesting the information of the health management device to an external server; and
receiving the information of the health management device from the external server.

10. A client device that recommends health management device by using connectivity information of the client device, the client device comprising:
a communicator capable of communicating with an external device; and
a controller, which recommends the health management device which is able to be connected to the client device, based on the connectivity information of the client device and information about health management device.

11. The client device of claim 10, further comprising an extractor, which extracts user's health information from the client device,
wherein the controller recommends only those health management devices which relate to a health condition of the user, from the health management devices which are able to be connected to the client device, by using the extracted user's health information.

12. The client device of claim 11, further comprising:
a transmitter, which requests the information of the health management device from an external server; and
a receiver, which receives the information of the health management device from the external server.

13. A non-transitory computer-readable recording medium having recorded thereon a computer program for executing the method of claim 1.

14. A non-transitory computer-readable recording medium having recorded thereon a computer program for executing the method of claim 7.
